# EUROPEAN PATENT SPECIFICATION

(11) **EP 4 399 059 B1**
(45) Date of publication and mention of the grant of the patent: **11.06.2025**
(21) Application number: 21773740.2
(22) Date of filing: 07.09.2021
(51) Int. Cl.: B25J 5/00, A61L 2/18, B08B 3/00, B25J 11/00, B25J 19/02, G01N 21/00

(54) **METHOD OF CLEANING REGION IN INDUSTRIAL ENVIRONMENT, CONTROL SYSTEM AND CLEANING SYSTEM**
VERFAHREN ZUM REINIGEN EINES BEREICHS IN EINER INDUSTRIELLEN UMGEBUNG, STEUERSYSTEM UND REINIGUNGSSYSTEM
PROCÉDÉ DE NETTOYAGE D'UNE ZONE DANS UN ENVIRONNEMENT INDUSTRIEL, SYSTÈME DE COMMANDE ET SYSTÈME DE NETTOYAGE

(43) Date of publication of application: 17.07.2024
(73) Proprietor: ABB SCHWEIZ AG, 5400 Baden (CH)
(72) Inventor: THOLENCE, Frederic, 431 63 Mölndal (SE); ERNLUND, Johan, 725 96 Västerås (SE); LI, Shanghua, 723 48 Västerås (SE)
(74) Representative: Kransell & Wennborg KB
(86) International application number: PCT/EP2021/074614
(87) International publication number: WO 2023/036405

(56) References cited:
- US-A1- 2012 223 216
- US-A1- 2020 397 936
- US-A1- 2021 038 041
- US-A1- 2021 161 351
- US-B1- 11 097 414

## Description

### Technical Field

The present disclosure generally relates to cleaning of industrial environments. In particular, a method of cleaning a region in an industrial environment using at least one industrial robot, a control system for controlling cleaning of a region in an industrial environment using at least one industrial robot, and a cleaning system comprising at least one industrial robot, are provided.

### Background

In the food and beverage industry, cleanliness is a key concern. Any sanitary problem in a food and beverage factory might result in catastrophic consequences. For this reason, comprehensive cleaning measures are often taken in such factories. The cleaning is often performed manually today where trained personnel apply different washdown chemicals. The manual cleaning instructions are typically extremely thorough, resulting in long cleaning times, high costs and a high environmental impact. The final result of the cleaning process is normally assessed visually and by manually taking samples at some points of the factory. The personnel performing the cleaning and validation of the cleaning is often difficult to recruit due to the difficulty of the tasks and the conditions of the work environment. The presence of humans close to a production line might also be a source of contamination.

CN 109452910 A discloses a floor sweeping robot containing chemical liquid.

US 2012223216 A1 discloses a sterilization system including a self-propelled robotic mobile platform for locating and eradicating infectious bacterial and virus strains on floors using one or more ultraviolet light sources. A controller allows the system to adjust the quantity of ultraviolet light received by a surface. The mobile platform may include a sensor capable of detecting fluorescence of biological contaminants irradiated with ultraviolet light to locate contaminated areas. The sterilization system is not suitable for washing an industrial environment, such as a food and beverage factory, and does not describe an efficient application of cleaning media.

US 11 097 414 B1 discloses a method of cleaning a region in an industrial environment using at least one industrial robot carrying a cleaning device, the method comprising: providing sensor data identifying one or more surfaces in the region touched by an actor; and performing a cleaning process to clean the region, the cleaning process comprising applying cleaning media from the cleaning device to the one or more surfaces, and the cleaning process being performed based on the sensor data.

### Summary

One object of the present disclosure is to provide a method of cleaning a region in an industrial environment, which method enables a higher level of cleanliness.

A further object of the present disclosure is to provide a method of cleaning a region in an industrial environment, which method is cost-efficient.

A still further object of the present disclosure is to provide a method of cleaning a region in an industrial environment, which method enables an efficient cleaning.

A still further object of the present disclosure is to provide a method of cleaning a region in an industrial environment, which method solves several or all of the foregoing objects in combination.

A still further object of the present disclosure is to provide a control system for controlling cleaning of a region in an industrial environment, which control system solves one, several or all of the foregoing objects.

A still further object of the present disclosure is to provide a cleaning system solving one, several or all of the foregoing objects.

According to a first aspect, there is provided a method of cleaning a region in an industrial environment using at least one industrial robot, the method comprising obtaining radiation from the region by means of a sensor device; providing sensor data based on the radiation, the sensor data being indicative of a cleanliness of one or more surfaces in the region; and performing a cleaning process to clean the region, the cleaning process comprising applying cleaning media from a cleaning device to the one or more surfaces, and the cleaning process being performed based on the sensor data, wherein the industrial robot carries the sensor device and/or the cleaning device.

By performing the cleaning process based on the sensor data, the cleaning process can be adapted to the cleanliness. The cleaning of the industrial environment is thereby made more efficient. It can for example be avoided that the cleaning process is performed on surfaces without a need for cleaning. Alternatively, such surfaces may be cleaned at a reduced intensity (e.g. during a shorter time and/or with a reduced amount of cleaning media). In this way, the cleaning time, water and the cleaning media can be more efficiently utilized.

Furthermore, by utilizing radiation to provide the sensor data indicative of the cleanliness of the one or more regions, the need to take physical contamination samples from the one or more regions is avoided. The radiation may be reflective radiation from the one or more regions.

Each of the at least one industrial robot may comprise a base and a manipulator movable relative to the base. The manipulator may be a serial manipulator comprising three or more axes, such as six or seven axes. At least one of the sensor device, the cleaning device and the one or more surfaces may be carried by one of the at least one industrial robot, such as by the manipulator thereof.

The industrial robot may be mobile or stationary. A stationary robot may comprise a stationary base where the manipulator is movable relative to the stationary base. Correspondingly, a mobile robot may comprise a mobile base where the manipulator is movable relative to the mobile base. The mobile base may be driven by a traction arrangement as described herein.

The sensor device may be carried by one of the at least one industrial robot or by a human, or may be stationary. In case the sensor device is carried by one of the at least one industrial robot, the cleaning device may be carried by the same industrial robot, may be stationary, may be carried by another of the at least one industrial robot, or may be carried by a human. In case the sensor device is stationary, the sensor device can be integrated into machines or other equipment in the industrial environment.

Alternatively, or in addition, the cleaning device may be carried by one of the at least one industrial robot or by a human, or may be stationary. In case the cleaning device is carried by one of the at least one industrial robot, the sensor device may be carried by the same industrial robot, may be stationary, may be carried by another of the at least one industrial robot, or may be carried by a human. In case the cleaning device is stationary, the cleaning device can be integrated into machines or other equipment in the industrial environment.

The cleanliness may be associated with an amount of contamination, such as an amount of bacteria, and/or a type of contaminants, on the one or more surfaces.

The cleaning media may comprise one or more cleaning detergents, one or more chemicals and/or one or more sanitizers. The cleaning media may additionally comprise water.

The sensor data may be provided by the sensor device or by a control system in signal communication with the sensor device. The sensor data may indicate a presence of contaminants in the environment. Optionally, the sensor data may also indicate a type of contaminants. The sensor device may however be configured to detect only the presence, and an optional amount of, one or a few types of contaminants and communicate this information as sensor data. A high amount of contaminants may correspond to a low cleanliness value and vice versa.

The sensor data may optionally also contain position information of the contaminants. Thus, the sensor device may communicate sensor data containing such position information. If such position information is not provided by the sensor device, the industrial robot carrying the sensor device may determine the position information and add this to the sensor data. In this case, the position information may be determined by the industrial robot based on a position of the manipulator when the radiation is received by the sensor device.

The sensor device may for example comprise a laser-induced breakdown spectroscopy (LIBS) sensor, a Fourier-transform infrared spectroscopy (FTIR) sensor, a Raman spectroscopy sensor, a biosensor, such as an optoacoustic biosensor and/or an optical biosensor. An optical biosensor may for example combine an optical effect and a thermal effect to reliably detect contaminants, such as pathogens. By obtaining radiation from the region, the sensor device can be a noninvasive or contactless sensor device. The sensor device may comprise a plurality of different types of sensors, e.g. to detect different types of contaminants.

The industrial environment may for example be a food manufacturing environment, a beverage manufacturing environment, a chemical product manufacturing environment, a pharmaceutical product manufacturing environment, or an agricultural product manufacturing environment. The industrial environment may comprise at least one handling tool for handling produced items, such as a cutting tool for cutting meat. Alternatively, or in addition, the industrial environment may comprise at least one packaging machine for packaging produced items.

The method may be carried out with a cleaning system according to the present disclosure.

The cleaning process may be performed using one or more cleaning parameters. Examples of such cleaning parameters comprise pressure of the cleaning media, temperature of the cleaning media, concentration of the cleaning media (e.g. when solved in water), and time of application of the cleaning media to the one or more surfaces.

The method may further comprise determining a cleanliness value based on the sensor data. In this case, the cleaning process may be performed based on the cleanliness value.

The method may further comprise determining, based on the sensor data, whether the cleaning process should be performed. In this way, it can accurately be determined when it is time to perform the cleaning process. Being able to monitor when it is time to clean the region enables substantial cost savings.

The method according to this variant may comprise determining an actual cleanliness value based on the sensor data and comparing the actual cleanliness value with a reference cleanliness value. As long as the actual cleanliness value is higher than the reference cleanliness value, the cleaning process may be postponed. When the cleanliness value is lower than the reference cleanliness value, the cleaning process may be performed. The method may thus comprise monitoring contaminants on the one or more surfaces by means of the sensor device.

The method may further comprise obtaining validation radiation from the region by means of the sensor device; generating validation sensor data based on the radiation; and evaluating the cleaning process based on the validation sensor data. The validation radiation may be obtained continuously or intermittently. Thus, the quality of the cleaning process can be monitored continuously or periodically. By means of the validation sensor data, feedback regarding the cleanliness is provided. It can then be determined whether or not the cleaning process has provided a targeted cleanliness. This contributes to a more efficient cleaning and to a reduction of used cleaning media and water.

Validation of the cleaning process is a powerful tool to secure the success of the cleaning process. By means of the validation, patterns of contamination developments in the region can be detected and a subsequent cleaning process can thereby be made more proactive. The evaluation of the cleaning process based on the further sensor data may be made according to a predefined criteria. The cleaning process can thereby be quality assured.

The method may further comprise selecting a type of cleaning media to be applied during the cleaning process based on the sensor data. This enables a most suitable cleaning media to be selected. As a consequence, cleaning efficiency is improved and costs are reduced. Two cleaning media may contain the same chemical but may differ by a concentration thereof.

The method may further comprise determining, based on the sensor data, a type of contaminant in the region. In this case, the method may further comprise performing the cleaning process based on the type of contaminant. Alternatively, or in addition, the method may further comprise selecting a type of cleaning media to be applied during the cleaning process based on the type of contaminant. In these ways, the cleaning process can be made even more efficient. The application of the cleaning media may be preceded by application of hot water, succeeded by application of cold water, and/or succeeded by application of hot water.

The method may further comprise determining a distribution of contaminants in the region based on the sensor data; and performing the cleaning process based on the distribution. By controlling the cleaning process based on the distribution of contaminants in the region, in contrast to cleaning the entire region, the cleaning process is made more efficient. For example, the method enables performance of the cleaning process locally, when needed.

The method may further comprise evaluating a surface property of the one or more surfaces in the region based on the sensor data. In this case, the method may further comprise performing the cleaning process based on the surface property. The surface property may indicate a type of material of the surface and/or a degree of wear of the surface.

In some machine process within the food and beverage industry, such as when cutting meat, the cleaning media contributes more to the wear than the process itself of the machine. When the one or more surfaces are worn, the cleanliness might be reduced. It is therefore highly valuable to evaluate the surface properties of the surfaces and to take necessary action at an early stage. Parts comprising soft materials, such as polymers and elastomers, parts comprising painted surfaces, and sealing parts, might particularly be subjected to wear by some cleaning media. It is therefore valuable to be able to not apply an excessive amount of cleaning media to such parts.

By evaluating the surface property, wear, scratches, pits from rust, corrosion and other damages to the one or more surfaces can be identified with the same means as used for providing the sensor data, i.e. the sensor device. The sensor data can thus provide information both regarding cleanliness and regarding surface properties. By performing the cleaning process and evaluating the surface properties based on the sensor data in parallel, the method is highly efficient.

The method according to this variant may comprise determining an actual surface property value based on the sensor data and comparing the actual surface property value with a reference surface property value. If the actual surface property value is worse than (e.g. lower than) the reference surface property value, the method may initiate a countermeasure, such as an increase in cleaning time and/or a maintenance operation. The initiation of the maintenance operation may comprise issuing an alarm and/or interrupting the cleaning process.

If the time to obtain a targeted level of cleanliness is too long, there might be a risk that the surface is damaged. By evaluating the surface property, the relevant cleaning parameters can be adjusted for future cleaning processes and the cleaning process can be adjusted accordingly for other surfaces of the same material.

The cleaning process may be improved by one or more machine learning algorithms. In this way, the machine learning algorithm can learn where contaminants often occur, how to improve cleanliness, how to reduce consumption of cleaning media and/or how to reduce cleaning time. Among these conditions, the cleanliness condition may have the highest priority. For example, one machine learning algorithm may build a model of the cleaning process using the sensor data, the surface properties, the cleanliness value and/or the cleaning parameters as training data. In this way, the cleaning system can be trained to optimize the cleaning performance.

The sensor device may be carried by one of the at least one industrial robot. The sensor device may be carried by the manipulator. The method may comprise moving the sensor device by the manipulator to the one or more surfaces.

The cleaning device may be carried by one of the at least one industrial robot. The cleaning device may be carried by the manipulator. The method may comprise moving the cleaning device by the manipulator to the one or more surfaces.

Due to the one or more manipulators, agile, dexterous and accurate movements of the sensor device and/or the cleaning device are enabled. The sensor device and/or the cleaning device may be moved rapidly in three dimensions and can reach areas that are difficult to reach by humans. Besides, the use of one or more manipulators allows for exhaustive and reproducible coverage of the surfaces to be cleaned. The cleaning performance is thereby improved.

One of the at least one industrial robot may be a mobile robot having a traction arrangement. The traction arrangement may be configured to move a base of the mobile robot over a base surface, such as a floor. The method may comprise moving the mobile robot by the traction arrangement in proximity to the one or more surfaces.

The traction arrangement may comprise a plurality of wheels. The wheels may drive a base in the form of a platform. At least one of the wheels may be a driving wheel and at least one of the wheels may be a steering wheel. The wheels may provide two or three degrees of freedom of the base on the base surface. Alternatively, the traction arrangement may be a linear traction arrangement, such as a conveyor belt.

The mobile robot and its manipulator may be moved autonomously. The method thereby enables an autonomous provision of sensor data indicative of the cleanliness.

The method may further comprise providing a map representing the industrial environment, and mapping the sensor data in the map. The map may be a grid-based map. In this case, the sensor data, and/or a cleanliness value determined based on sensor data, may be associated with one, several or all cells of the grid-based map. The map may be three-dimensional. By mapping the sensor data in a three-dimensional map and controlling the cleaning device by means of a manipulator of a mobile robot, the cleaning process can be made more efficient.

Each mobile robot may be a collaborative robot. The method can thereby be carried out in human populated environment without needing to interrupt any process carried out by nearby humans.

The method may further comprise controlling the traction arrangement to move the mobile robot to dock to a docking station; and supplying cleaning media from the docking station to one or more tanks of the mobile robot. In this way, the use of external hoses can be avoided.

The method may further comprise electrically charging the mobile robot from the docking station when the mobile robot is docked to the docking station. In this way, the use of external cables can be avoided.

The docking station may comprise a docking station cleaning robot. The docking station cleaning robot may be configured to clean the mobile robot when docked to the docking station using cleaning media from one or more tanks of the docking station.

The method may further comprise communicating management data to a remote management system, the management data being associated with the sensor data and/or data associated with the cleaning process. The management system may receive such management data from a plurality of different industrial environments. In this way, the cleaning process can be improved using knowledge from other industrial environments.

According to a second aspect, there is provided a control system for controlling cleaning of a region in an industrial environment using at least one industrial robot, the control system comprising at least one data processing device and at least one memory having at least one computer program stored thereon, the at least one computer program comprising program code which, when executed by the at least one data processing device, causes the at least one data processing device to perform the steps of commanding a sensor device to obtain radiation from the region; providing sensor data based on the radiation, the sensor data being indicative of a cleanliness of one or more surfaces in the region; and controlling performance of a cleaning process to clean the region, the cleaning process comprising applying cleaning media from a cleaning device to the one or more surfaces, and the cleaning process being controlled based on the sensor data. The at least one computer program may further comprise program code which, when executed by the at least one data processing device, causes the at least one data processing device to perform, or command performance of, any step of the method of the first aspect. The one or more machine learning algorithms may be implemented in the control system.

According to a third aspect, there is provided a cleaning system comprising the control system according to the second aspect; the at least one industrial robot; the sensor device; and the cleaning device. The cleaning system may be of any type as described herein. The cleaning system may be configured to carry out any variant of the method according to the first aspect. The cleaning system may comprise a robot fleet of a plurality of industrial robots.

One of the at least one industrial robot may be a mobile robot having a traction arrangement. In this case, the cleaning device may be carried by the mobile robot. The mobile robot may comprise a base and a manipulator movable relative to the base. The sensor device and/or the cleaning device may be carried by the manipulator.

The cleaning system may further comprise a docking station. In this case, the at least one mobile robot and the docking station may be configured such that the at least one mobile robot can dock to the docking station and receive cleaning media therefrom. The docking station may also be configured to supply water, compressed air and/or electric power to the at least one mobile robot.

### Brief Description of the Drawings

Further details, advantages and aspects of the present disclosure will become apparent from the following description taken in conjunction with the drawings, wherein:
- Fig. 1:: schematically represents a cleaning system comprising an industrial robot;
- Fig. 2:: schematically represents a top view of a one example of a cleaning system in an industrial environment;
- Fig. 3:: schematically represents a top view of a further example of a cleaning system in the industrial environment;
- Fig. 4:: schematically represents a top view of a further example of a cleaning system in the industrial environment;
- Fig. 5:: schematically represents a top view of a further example of a cleaning system in the industrial environment; and
- Fig. 6:: schematically represents a top view of a further example of a cleaning system in the industrial environment.

### Detailed Description

In the following, a method of cleaning a region in an industrial environment using at least one industrial robot, a control system for controlling cleaning of a region in an industrial environment using at least one industrial robot, and a cleaning system comprising at least one industrial robot, will be described. The same or similar reference numerals will be used to denote the same or similar structural features.

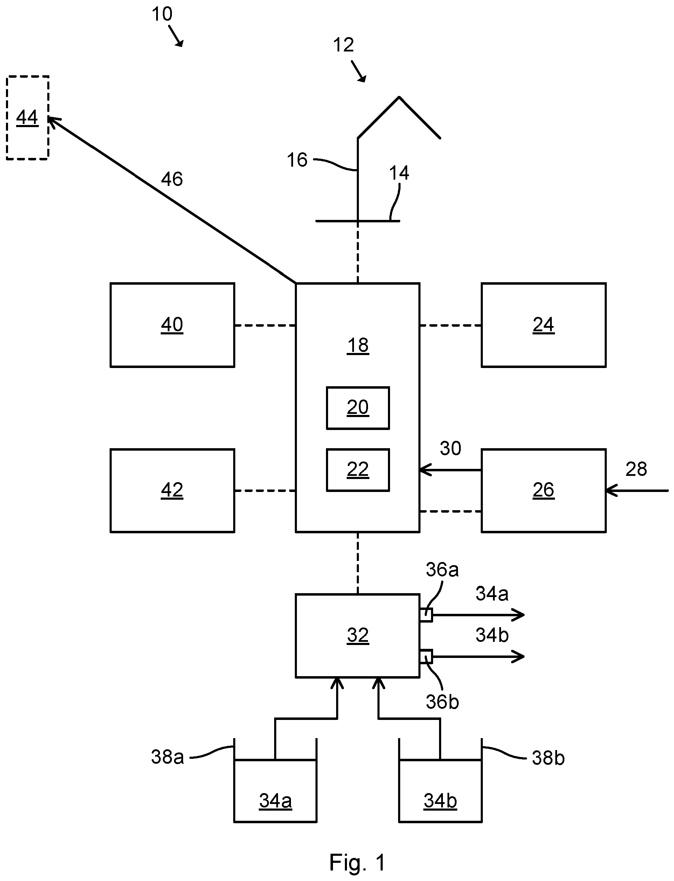

Fig. 1 schematically represents a cleaning system 10. The cleaning system 10 comprises an industrial robot 12. The industrial robot 12 comprises a base 14 and a manipulator 16 movable relative to the base 14. The manipulator 16 may be a serial manipulator comprising three or more axes, such as six or seven axes.

The cleaning system 10 further comprises a control system 18. The control system 18 of this example comprises a data processing device 20 and a memory 22. The memory 22 has a computer program stored thereon. The computer program comprises program code which, when executed by the data processing device 20, causes the data processing device 20 to perform, or command performance of, various steps as described herein. The control system 18 is in signal communication with the industrial robot 12 and here controls the industrial robot 12.

The cleaning system 10 of this example further comprises a traction arrangement 24. The traction arrangement 24 is configured to move the base 14 over a base surface, such as a floor. To this end, the traction arrangement 24 may comprise a plurality of wheels. The traction arrangement 24 is in signal communication with the control system 18 for being controlled by the control system 18.

The cleaning system 10 further comprises a sensor device 26. The sensor device 26 of this example is configured to irradiate a surface and to receive reflected radiation 28 from the surface. The sensor device 26 of the invention is further configured to provide sensor data 30 indicative of a cleanliness of the surface based on the radiation 28. The sensor device 26 may for example comprise an optical sensor, such as a laser-induced breakdown spectroscopy (LIBS) sensor. The sensor device 26 can be carried by the manipulator 16. The control system 18 is in signal communication with the sensor device 26 and can receive the sensor data 30 from the sensor device 26. The sensor device 26 may optionally comprise a plurality of different types of sensors. The control system 18 is configured to control the sensor device 26, e.g. to control the sensor device 26 to irradiate an object for receiving the radiation 28.

The cleaning system 10 further comprises a cleaning device 32. The cleaning device 32 may comprise one or more nozzles for applying cleaning media 34a and 34b. The cleaning device 32 of this specific example comprises a first nozzle 36a for applying a first type of cleaning media 34a and a second nozzle 36b for applying a different second type of cleaning media 34b. The cleaning media 34a and 34b may for example be different types of detergents or sanitizers. The cleaning media 34a and 34b may differ by concentration and/or by type of chemical.

The cleaning system 10 of this example further comprises a first tank 38a containing the first cleaning media 34a and a second tank 38b containing the second cleaning media 34b. The cleaning media 34a and 34b may optionally be diluted with water. To this end, the cleaning system 10 may comprise a dedicated mixing system (not shown) to adjust a concentration of the cleaning media 34a and 34b prior to their application. The cleaning device 32, the tanks 34a and 34b and the optional mixing system can be carried by the industrial robot 12, such as by the traction arrangement 24 thereof. The cleaning device 32 is in signal communication with the control system 18 for being controlled by the control system 18.

The cleaning system 10 of this example further comprises a docking station 40. The docking station 40 is configured to supply cleaning media 34a and 34b to the respective tanks 38a and 38b, and is configured to supply electric power to the industrial robot 12, such as to the traction arrangement 24. The docking station 40 is in signal communication with the control system 18 for being controlled by the control system 18.

The cleaning system 10 of this example further comprises a docking station cleaning robot 42. The docking station cleaning robot 42 of this example comprises a stationary base and a manipulator movable relative to the stationary base. The docking station cleaning robot 42 is in signal communication with the control system 18 for being controlled by the control system 18.

Fig. 1 further shows a remote management system 44. The management system 44 may be implemented on the Internet. The control system 18 is in signal communication with the management system 44. The control system 18 is configured to send management data 46 to the management system 44.

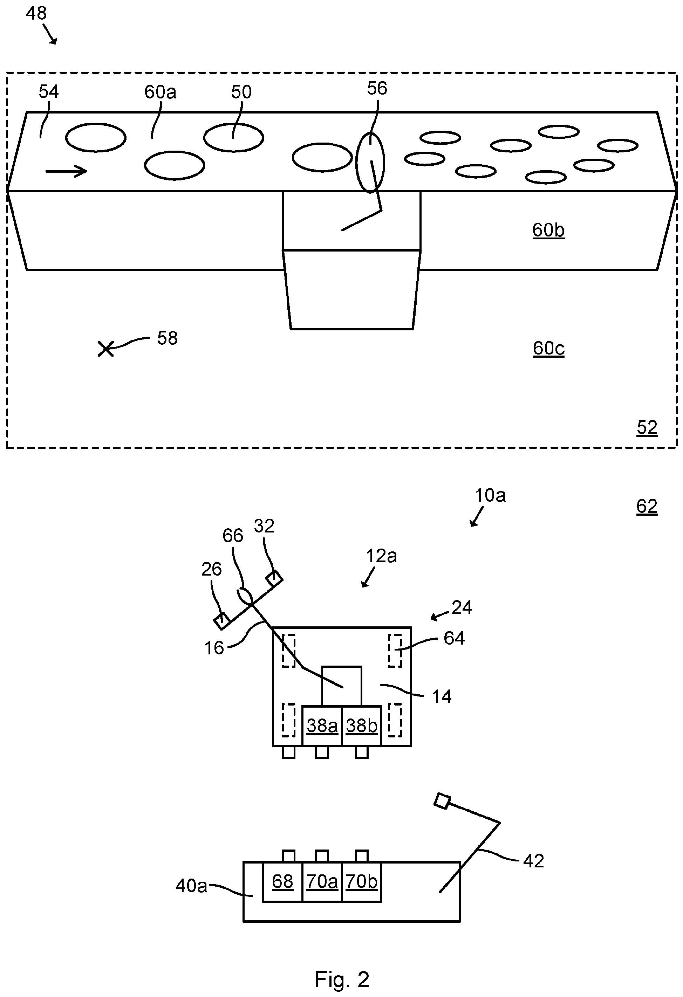

Fig. 2 schematically represents a top view of one example of a cleaning system 10a in an industrial environment 48. The cleaning system 10a corresponds to the cleaning system 10 in Fig. 1. The industrial environment 48 may be an environment where ingestible items 50 are produced, such as a factory for producing food and/or beverages. The industrial environment 48 comprises a region 52 through which the ingestible items 50 pass. The region 52 of this example comprises a conveyor 54 for transporting the ingestible items 50 and a cutting tool 56, such as a knife or a saw blade, for cutting the ingestible items 50. The cutting tool 56 is one example of a handling tool for handling the ingestible items 50. The ingestible items 50 are here exemplified as pieces of meat that are cut into smaller pieces by the cutting tool 56.

Fig. 2 further schematically illustrates contaminants 58 present in the region 52. The contaminants 58 may originate from the processing of the ingestible items 50 or from different sources. Examples of contaminants 58 comprise fat, oils, blood, proteins, starches, soils, deposits, algae, fungi and/or lime scale.

While some of these contaminants 58 might cause a health risk and should be sanitized, other contribute to a poor visual appearance that cannot be accepted in the region 52. Besides, some of these contaminants 58 may absorb other soils and microorganisms and become a hazard in the long term.

The region 52 of the industrial environment 48 has high sanitary requirements. The region 52 of this specific and illustrative example comprises three different surfaces: a first surface 60a on top of the conveyor 54, a second surface 60b on a vertical side of the conveyor 54, and a third surface 60c on a floor 62 below the conveyor 54. A unique hygiene level may be specified for each of the surfaces 60a-60c. The first surface 60a may be a primary hygiene zone, where the hygienic requirement is the highest since the ingestible items 50 are in direct contact therewith. The second surface 60b may be a so-called splash zone where splashes of the ingestible items 50 can adhere. The second surface 60b should be cleaned in order to prevent formation of nests of contaminants 58 thereon, but the hygienic requirement may be somewhat lower than for the first surface 60a. The hygienic requirement of the third surface 60c may be further lower than for the second surface 60b.

The industrial robot 12 is here exemplified as a mobile robot 12a. The mobile robot 12a of this example is a collaborative robot that is constructed to work in collaboration with humans and that is incapable of injuring humans. In Fig. 2, it can be seen that the traction arrangement 24 of this example comprises a plurality of wheels 64 for driving the base 14 of the mobile robot 12a over the floor 62.

In the cleaning system 10a, the manipulator 16 of the mobile robot 12a carries both the sensor device 26 and the cleaning device 32, here at a distal end of the manipulator 16. The manipulator 16 of this example also comprises a gripper 66 or other type of end effector for performing additional tasks. The mobile robot 12a also carries the first and second tanks 38a and 38b containing the first cleaning media 34a and the second cleaning media 34b, respectively.

Fig. 2 further shows a docking station 40a, which is one example of the docking station 40 in Fig. 1. The docking station 40a of this example comprises a mains supply 68, a first cleaning media supply 70a for supplying the first cleaning media 34a and a second cleaning media supply 70b for supplying the second cleaning media 34b. Fig. 2 further shows the docking station cleaning robot 42 of the docking station 40a.

The mobile robot 12a can drive over the floor 62 to dock to the docking station 40a. When docked, an electric energy storage of the mobile robot 12a is electrically charged by the mains supply 68 and is refilled with cleaning media 34a and 34b therefrom. During this charging and refilling, the docking station cleaning robot 42 is controlled to wash the mobile robot 12a using one or both of the cleaning media 34a and 34b.

Before cleaning the region 52, the mobile robot 12a is driven towards the conveyor 54. The base 14 and/or the manipulator 16 is then controlled to move the sensor device 26 to scan the surfaces 60a-60c in a contactless manner. The mobile robot 12a may be controlled autonomously to perform this scanning. During the scanning, the sensor device 26 obtains radiation 28 from the respective surfaces 60a-60c and generates corresponding sensor data 30 indicative of the cleanliness thereof. The sensor device 26 may be configured to detect the presence of contaminants 58 and/or to detect an amount of contaminants 58 on the respective surfaces 60a-60c based on which corresponding cleanliness values are generated as sensor data 30. In the former case, the cleanliness value may be set low for surfaces 60a-60c where contaminants 58 are detected. In the latter case, the cleanliness value may be set relatively low for surfaces 60a-60c where relatively high amounts of contaminants 58 are detected and vice versa.

Since the control system 18 controls the mobile robot 12a, the control system 18 is aware of the position of the sensor device 26 at each time. The sensor data 30 can thereby also contain positional information regarding the position of the sensor device 26 when receiving the radiation 28. In this way, a distribution of contaminants 58 in the region 52 can efficiently be provided as sensor data 30. The distribution of contaminants 58 may be stored in a three-dimensional grid-based map for a subsequent cleaning process.

The sensor data 30 is evaluated by the control system 18. The control system 18 initially determines based on the sensor data 30 whether or not a cleaning process for the region 52 is necessary. To this end, the control system 18 may compare an actual cleanliness value determined based on, or constituted by, the sensor data 30 with a reference cleanliness value. The reference cleanliness value may be unique for each surface 60a-60c. If the region 52 is judged to be sufficiently clean, the mobile robot 12a may return to the docking station 40a. The mobile robot 12a may then return to the region 52 to make a new scan after a predetermined time period, or when commanded.

In case the control system 18 judges based on the sensor data 30 that the region 52 is not sufficiently clean, the cleaning process is initiated. The mobile robot 12a then performs the cleaning process based on the sensor data 30. During the cleaning process, the base 14 and/or the manipulator 16 is controlled to move the cleaning device 32 towards the surfaces 60a-60c and is controlled to flush cleaning media 34a and 34b onto the surfaces 60a-60c. By using the sensor data 30 as feedback for the cleaning process, the cleaning process can be improved. Also the cleaning process can be performed autonomously by the mobile robot 12a.

In one example, the sensor device 26 is configured to provide sensor data 30 indicating a type of contaminant 58. In this case, the first cleaning media 34a may be selected for application to a surface 60a-60c where a first type of contaminant 58 has been detected and the second cleaning media 34b may be selected for application to a surface 60a-60c where a different second type of contaminant 58 has been detected. Other parameters of the cleaning process may alternatively or in addition be controlled based on the type of the contaminants 58.

In a further example, the sensor device 26 is configured to provide sensor data 30 based on the radiation 28 indicating a surface property of each of the surfaces 60a-60c. The surface property may indicate a condition of the surface material. For example, by means of a laser-induced breakdown spectroscopy (LIBS) sensor, not only contaminations, but also surface properties can be determined. The surface property of each surface 60a-60c may also be taken into account during the cleaning process. For example, a more careful cleaning may be performed on delicate materials or on worn materials. If a poor surface property is detected, the cleaning process may be interrupted and an alarm may be issued.

During the cleaning process, the control system 18 may also control cleaning parameters associated therewith, such as a pressure of the cleaning media 34a and 34b, a temperature of the cleaning media 34a and 34b, a concentration of the cleaning media 34a and 34b, and a time of application of the cleaning media 34a and 34b to the respective surface 60a-60b, based on the sensor data 30. The mobile robot 12a enables automated inspection and cleaning of many different regions in the industrial environment 48, in addition to the region 52.

After the cleaning process, the mobile robot 12a may again scan the region 52 with the sensor device 26 to validate the cleaning process. In this case, the sensor device 26 is controlled to obtain validation radiation 28 from the region 52 and to forward corresponding validation sensor data 30 to the control system 18. The process of obtaining validation radiation 28 and the validation sensor data 30 corresponds to the initial scan before the cleaning process. The cleaning process is then evaluated based on the validation sensor data 30. For example, cleanliness values before and after the cleaning process can be compared to evaluate the efficiency of the cleaning process. Correspondingly, surface property values before and after the cleaning process can be compared to evaluate any potential wear caused by the cleaning process. In this way, the cleaning process can be repeatedly optimized, e.g. by selecting the most appropriate cleaning media 34a and 34b for a respective surface 60a-60c. Based on the validation sensor data 30, areas of poor hygienic design, areas where contaminations often occurs, and areas containing resistant contaminants 58 can also be pinpointed by the control system 18 and/or by the management system 44. Besides, an early identification of new types of contaminants 58 is enabled.

The cleaning process can repeatedly be improved based on the sensor data 30 and the cleaning process parameters over several cleaning cycles. The sensor data 30 and the cleaning process parameters may for example be used as training data for a machine learning algorithm implemented in the control system 18. In this way, the cleaning process can efficiently be optimized for subsequent cleaning processes.

Management data 46 associated with the cleaning process, such as the sensor data 30 and/or various cleaning parameters, are also communicated to the remote management system 44. The remote management system 44 receives management data 46 from a plurality of different cleaning systems and can centrally provide instructions to the cleaning system 10a regarding various cleaning requirements and/or instructions. The management system 44 can control a fleet of industrial robots 12 to coordinate cleaning of the entire industrial environment 48.

After the validation scan, the mobile robot 12a may return to dock to the docking station 40a if no complementary cleaning measures are needed. The mobile robot 12a is then electrically recharged and refilled with cleaning media 34a and 34b. Moreover, the docking station cleaning robot 42 is controlled to wash the mobile robot 12a. After cleaning, recharging and refilling of cleaning media 34a and 34b, the mobile robot 12a may again patrol the region 52 to evaluate the cleanliness thereof.

The docking station 40a may alternatively be provided immediately adjacent to the surfaces 60a-60c, here adjacent to the conveyor 54, such as under the conveyor 54. In this way, the mobile robot 12a can remain connected to the docking station 40a during the cleaning process for a continuous supply of electric power and cleaning media 34a and 34b therefrom.

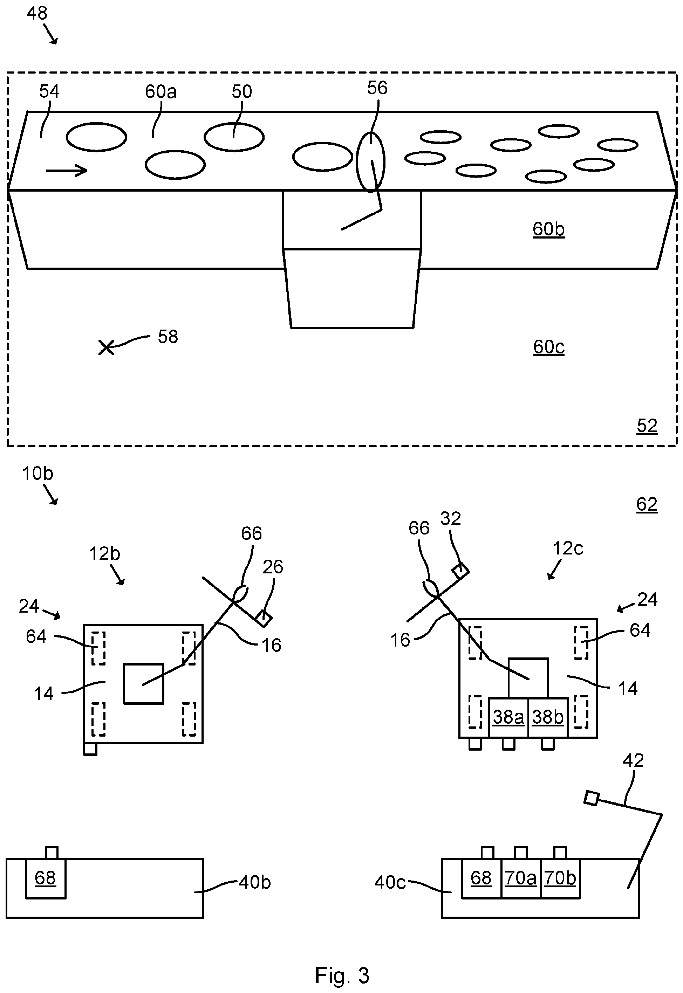

Fig. 3 schematically represents a top view of a further example of a cleaning system 10b in the industrial environment 48. Mainly differences with respect to Fig. 2 will be described.

The cleaning system 10b comprises a first mobile robot 12b and a second mobile robot 12c. The cleaning system 10b thus corresponds to the cleaning system 10 in Fig. 1 except for comprising two mobile robots 12b and 12c. The first mobile robot 12b comprises a manipulator 16 carrying the sensor device 26. The second mobile robot 12c comprises a manipulator 16 carrying the cleaning device 32. The second mobile robot 12c also carries the first and second tanks 38a and 38b.

The cleaning system 10b further comprises a first docking station 40b and a second docking station 40c. The first docking station 40b comprises a mains supply 68 for electrically charging the first mobile robot 12b when docked thereto. The second docking station 40c comprises a mains supply 68 for electrically charging the second mobile robot 12c, and the first and second cleaning media supplies 70a and 70b for supplying the first and second cleaning media 34a and 34b, respectively.

In this example, the first mobile robot 12b performs the scanning of the region 52 to obtain the sensor data 30 therefrom and the second mobile robot 12c performs the cleaning process. The control system 18 controls both the first and second mobile robots 12b and 12c.

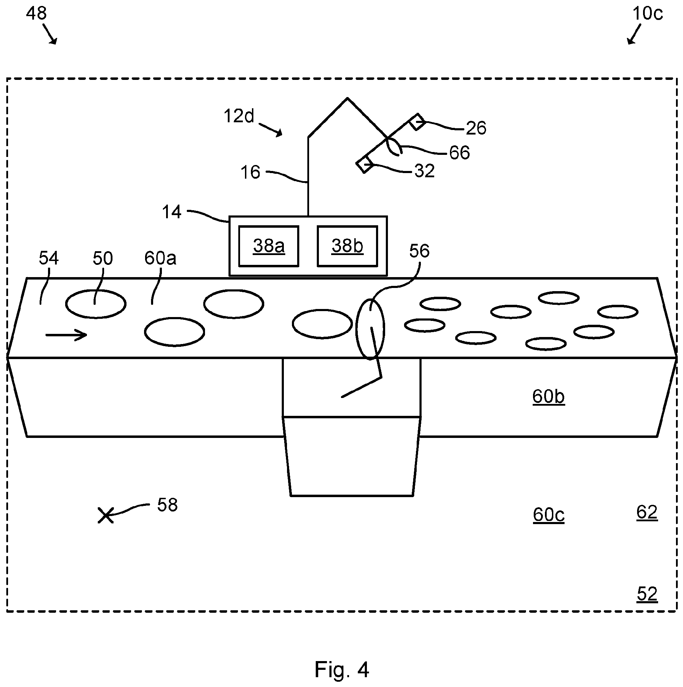

Fig. 4 schematically represents a top view of a further example of a cleaning system 10c in the industrial environment 48. Mainly differences with respect to Fig. 2 will be described.

The cleaning system 10c comprises a stationary robot 12d. The cleaning system 10c thus corresponds to the cleaning system 10 in Fig. 1 except for comprising the stationary robot 12d, no docking station 40, no docking station cleaning robot 42 and no traction arrangement 24.

The stationary robot 12d of this example comprises a stationary base 14 containing the first and second tanks 38a and 38b and a manipulator 16 movable relative to the base 14. The manipulator 16 in Fig. 4 carries both the sensor device 26 and the cleaning device 32. The stationary robot 12d is also configured to scan each of the surfaces 60a-60c and to clean the same, substantially as described in connection with Fig. 2.

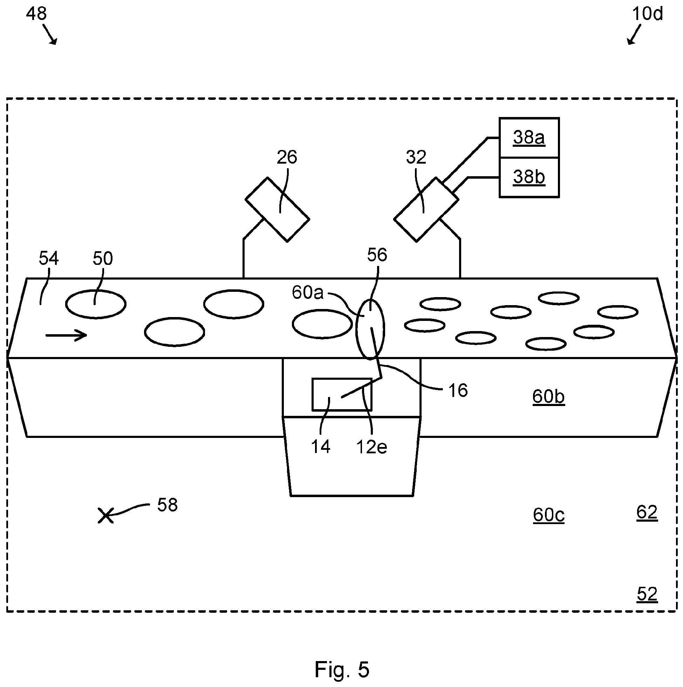

Fig. 5 schematically represents a top view of a further example of a cleaning system 10d in the industrial environment 48. Mainly differences with respect to Fig. 2 will be described.

The cleaning system 10d comprises a stationary robot 12e having a stationary base 14 and a manipulator 16 carrying the cutting tool 56. The cleaning system 10d corresponds to the cleaning system 10 in Fig. 1 except for comprising a stationary robot 12e, no docking station 40, no docking station cleaning robot 42 and no traction arrangement 24. Furthermore, the cleaning system 10d comprises a stationary sensor device 26 and a stationary cleaning device 32.

When not cutting the ingestible items 50, the cutting tool 56 can first be moved by the manipulator 16 of the stationary robot 12e to the sensor device 26. The sensor device 26 can thereby obtain sensor data 30 based on radiation 28 from the surface 60a of the cutting tool 56. The stationary robot 12e can then be moved relative to the cleaning device 32 for carrying out a cleaning process as described above, but where the cleaning device 32 is stationary and the surface 60a moves with respect to the same. By means of the stationary sensor device 26, a continuous monitoring of the conveyor 54 is possible.

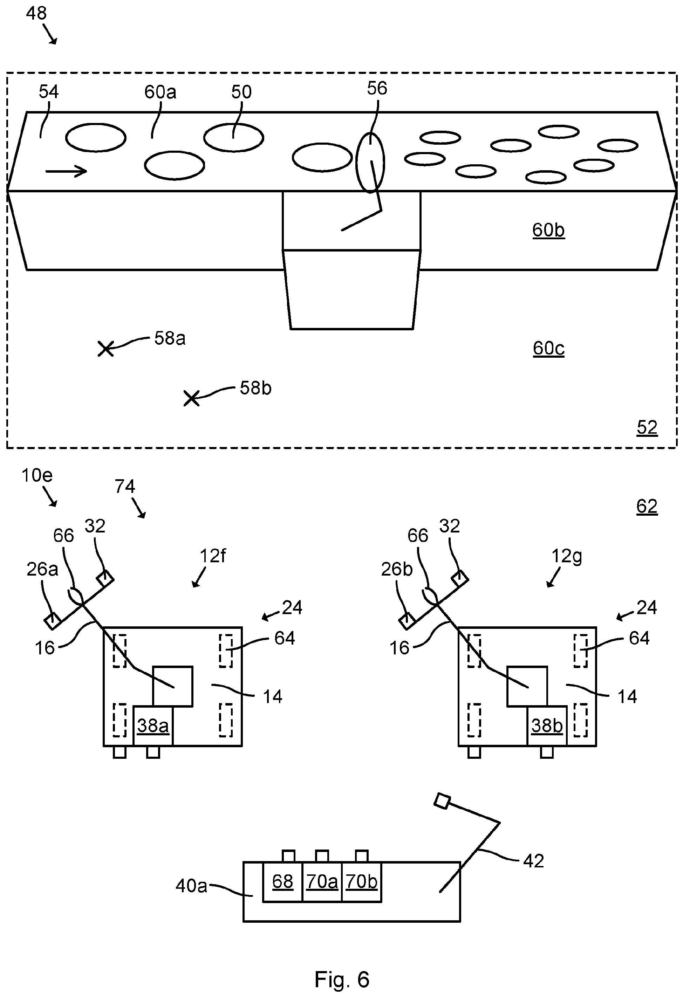

Fig. 6 schematically represents a top view of a further example of a cleaning system 10e in the industrial environment 48. Mainly differences with respect to Figs. 2 and 3 will be described.

The cleaning system 10e comprises a fleet 74 of mobile robots, here illustrated as a first mobile robot 12f and a second mobile robot 12g. The cleaning system 10e thus corresponds to the cleaning system 10 in Fig. 1 except for comprising two mobile robots 12f and 12g. The first mobile robot 12f comprises a manipulator 16 carrying a first sensor device 26a and a cleaning device 32 as described herein. The first mobile robot 12f carries the first tank 38a. The second mobile robot 12g comprises a manipulator 16 carrying a second sensor device 26b and a cleaning device 32 as described herein. The second mobile robot 12g carries the second tank 38b. Each of the sensor devices 26a and 26b is configured to obtain radiation 28 from the region 52 and to provide corresponding sensor data 30 indicative of a cleanliness of the surfaces 60a-60c. However, the first and second sensor devices 26a and 26b are of different types.

Fig. 6 further schematically illustrates two different types of contaminants 58a and 58b present in the region 52. The first mobile robot 12f is configured to detect a first type of contaminant 58a by means of the first sensor device 26a and to clean the first type of contaminant 58a by means of the first cleaning media 34a contained in the first tank 38a. The second mobile robot 12g is configured to detect a second type of contaminant 58b by means of the second sensor device 26b and to clean the second type of contaminant 58b by means of the second cleaning media 34b contained in the second tank 38b.

Each of the first and second mobile robots 12f and 12g is thus configured for a specific type of contamination 58a and 58b. The cleaning system 10e limits a risk of mixing the cleaning media 34a and 34b. Besides, the cleaning system 10e simplifies the design of the respective mobile robots 12f and 12g as they only need to be compatible with the respective cleaning media 34a and 34b.

While the present disclosure has been described with reference to exemplary embodiments, it will be appreciated that the present invention is not limited to what has been described above. For example, it will be appreciated that the dimensions of the parts may be varied as needed. Accordingly, it is intended that the present invention may be limited only by the scope of the claims appended hereto.

## Claims

1. A method of cleaning a region (52) in an industrial environment (48) using at least one industrial robot (12; 12a-12g), the method comprising:
- obtaining radiation (28) from the region (52) by means of a sensor device (26; 26a, 26b);
- providing sensor data (30) based on the radiation (28), the sensor data (30) being indicative of a cleanliness of one or more surfaces (60a-60c) in the region (52); and
- performing a cleaning process to clean the region (52), the cleaning process comprising applying cleaning media (34a, 34b) from a cleaning device (32) to the one or more surfaces (60a-60c), and the cleaning process being performed based on the sensor data (30),
wherein the industrial robot (12, 12a-12g) carries the sensor device (26; 26a, 26b) and/or the cleaning device (32).

2. The method according to claim 1, further comprising determining, based on the sensor data (30), whether the cleaning process should be performed.

3. The method according to any of the preceding claims, further comprising:
- obtaining validation radiation (28) from the region (52) by means of the sensor device (26; 26a, 26b);
- generating further sensor data (30) based on the validation radiation (28); and
- evaluating the cleaning process based on the validation sensor data (30).

4. The method according to any of the preceding claims, further comprising selecting a type of cleaning media (34a, 34b) to be applied during the cleaning process based on the sensor data (30).

5. The method according to any of the preceding claims, further comprising:
- determining a distribution of contaminants (58; 58a, 58b) in the region (52) based on the sensor data (30); and
- performing the cleaning process based on the distribution.

6. The method according to any of the preceding claims, further comprising:
- evaluating a surface property of the one or more surfaces (60a-60c) in the region (52) based on the sensor data (30); and
- performing the cleaning process based on the surface property.

7. The method according to any of the preceding claims, wherein one of the at least one industrial robot (12; 12a-12g) is a mobile robot (12a-12c; 12f, 12g) having a traction arrangement (24).

8. The method according to claim 7, further comprising:
- controlling the traction arrangement (24) to move the mobile robot (12a; 12c; 12f, 12g) to dock to a docking station (40; 40a-40c); and
- supplying cleaning media (34a, 34b) from the docking station (40; 40a-40c) to one or more tanks (38a, 38b) of the mobile robot (12a; 12c; 12f, 12g).

9. The method according to any of the preceding claims, further comprising communicating management data (46) to a remote management system (44), the management data (46) being associated with the sensor data (30) and/or associated with the cleaning process.

10. A control system (18) for controlling cleaning of a region (52) in an industrial environment (48) using at least one industrial robot (12; 12a-12g), the control system (18) comprising at least one data processing device (20) and at least one memory (22) having at least one computer program stored thereon, the at least one computer program comprising program code which, when executed by the at least one data processing device (20), causes the at least one data processing device (20) to perform the steps of:
- commanding a sensor device (26; 26a, 26b) to obtain radiation (28) from the region (52);
- providing sensor data (30) based on the radiation (28), the sensor data (30) being indicative of a cleanliness of one or more surfaces (60a-60c) in the region (52); and
- controlling performance of a cleaning process to clean the region (52), the cleaning process comprising applying cleaning media (34a, 34b) from a cleaning device (32) to the one or more surfaces (60a-60c), and the cleaning process being controlled based on the sensor data (30), wherein the industrial robot (12, 12a-12g) carries the sensor device (26; 26a, 26b) and/or the cleaning device (32).

11. A cleaning system (10; 10a-10e) comprising:
- the control system (18) according to claim 10;
- the at least one industrial robot (12; 12a-12g);
- the sensor device (26; 26a, 26b); and
- the cleaning device (32).

12. -The cleaning system (10; 10a-10e) according to claim 10, wherein one of the at least one industrial robot (12; 12a-12g) is a mobile robot (12a-12c; 12f, 12g) having a traction arrangement (24), and wherein the cleaning device (32) is carried by the mobile robot (12a; 12c; 12f, 12g).

13. -The cleaning system (10; 10a-10e) according to claim 12, further comprising a docking station (40; 40a-40c), wherein the mobile robot (12a; 12c; 12f, 12g) and the docking station (40; 40a-40c) are configured such that the at least one mobile robot (12a; 12c; 12f, 12g) can dock to the docking station (40; 40a-40c) and receive cleaning media (34a, 34b) therefrom.

## Patentansprüche

1. Verfahren zum Reinigen eines Bereichs (52) in einer industriellen Umgebung (48) unter Verwendung mindestens eines Industrieroboters (12; 12a-12g), wobei das Verfahren Folgendes umfasst:
- Erhalten von Strahlung (28) aus dem Bereich (52) mittels einer Sensorvorrichtung (26; 26a, 26b);
- Bereitstellen von Sensordaten (30) basierend auf der Strahlung (28), wobei die Sensordaten (30) eine Sauberkeit einer oder mehrerer Oberflächen (60a-60c) in dem Bereich (52) angeben; und
- Durchführen eines Reinigungsprozesses zum Reinigen des Bereichs (52), wobei der Reinigungsprozess das Anwenden von Reinigungsmedien (34a, 34b) von einer Reinigungsvorrichtung (32) auf die eine oder die mehreren Oberflächen (60a-60c) umfasst und der Reinigungsprozess basierend auf den Sensordaten (30) durchgeführt wird, wobei der Industrieroboter (12, 12a-12g) die Sensorvorrichtung (26; 26a, 26b) und/oder die Reinigungsvorrichtung (32) trägt.

2. Verfahren nach Anspruch 1, ferner umfassend das Bestimmen, basierend auf den Sensordaten (30), ob der Reinigungsprozess durchgeführt werden sollte.

3. Verfahren nach einem der vorhergehenden Ansprüche, das ferner Folgendes umfasst:
- Erhalten von Validierungsstrahlung (28) aus dem Bereich (52) mittels der Sensorvorrichtung (26; 26a, 26b);
- Erzeugen weiterer Sensordaten (30) basierend auf der Validierungsstrahlung (28); und
- Auswerten des Reinigungsprozesses auf der Grundlage der Validierungssensordaten (30).

4. Verfahren nach einem der vorhergehenden Ansprüche, ferner umfassend das Auswählen eines Typs von Reinigungsmedien (34a, 34b), die während des Reinigungsprozesses angewendet werden sollen, basierend auf den Sensordaten (30).

5. Verfahren nach einem der vorhergehenden Ansprüche, das ferner Folgendes umfasst:
- Bestimmen einer Verteilung von Verunreinigungen (58; 58a, 58b) in dem Bereich (52) basierend auf den Sensordaten (30); und
- Durchführen des Reinigungsprozesses basierend auf der Verteilung.

6. Verfahren nach einem der vorhergehenden Ansprüche, das ferner Folgendes umfasst:
- Auswerten einer Oberflächeneigenschaft der einen oder der mehreren Oberflächen (60a-60c) in dem Bereich (52) basierend auf den Sensordaten (30); und
- Durchführen des Reinigungsprozesses basierend auf der Oberflächeneigenschaft.

7. Verfahren nach einem der vorhergehenden Ansprüche, wobei einer des mindestens einen Industrieroboters (12; 12a-12g) ein mobiler Roboter (12a-12c; 12f, 12g) mit einer Traktionsanordnung (24) ist.

8. Verfahren nach Anspruch 7, das ferner Folgendes umfasst:
- Steuern der Traktionsanordnung (24), um den mobilen Roboter (12a; 12c; 12f, 12g) zu bewegen, um an eine Andockstation (40; 40a-40c) anzudocken; und
- Zuführen von Reinigungsmedien (34a, 34b) von der Andockstation (40; 40a-40c) zu einem oder mehreren Tanks (38a, 38b) des mobilen Roboters (12a; 12c; 12f, 12g).

9. Verfahren nach einem der vorhergehenden Ansprüche, ferner umfassend das Übermitteln von Verwaltungsdaten (46) an ein Fernverwaltungssystem (44), wobei die Verwaltungsdaten (46) mit den Sensordaten (30) assoziiert sind und/oder mit dem Reinigungsprozess assoziiert sind.

10. Steuersystem (18) zum Steuern der Reinigung eines Bereichs (52) in einer industriellen Umgebung (48) unter Verwendung mindestens eines Industrieroboters (12; 12a-12g), wobei das Steuersystem (18) mindestens eine Datenverarbeitungsvorrichtung (20) und mindestens einen Speicher (22) mit mindestens einem darauf gespeicherten Computerprogramm umfasst, wobei das mindestens eine Computerprogramm einen Programmcode umfasst, der, wenn er von der mindestens einen Datenverarbeitungsvorrichtung (20) ausgeführt wird, die mindestens eine Datenverarbeitungsvorrichtung (20) veranlasst, die folgenden Schritte durchzuführen:
- Anweisen einer Sensorvorrichtung (26; 26a, 26b), Strahlung (28) aus dem Bereich (52) zu erhalten;
- Bereitstellen von Sensordaten (30) basierend auf der Strahlung (28), wobei die Sensordaten (30) eine Sauberkeit einer oder mehrerer Oberflächen (60a-60c) in dem Bereich (52) angeben; und
- Steuern der Durchführung eines Reinigungsprozesses zum Reinigen des Bereichs (52), wobei der Reinigungsprozess das Anwenden von Reinigungsmedien (34a, 34b) von einer Reinigungsvorrichtung (32) auf die eine oder die mehreren Oberflächen (60a-60c) umfasst und der Reinigungsprozess basierend auf den Sensordaten (34a) gesteuert wird, wobei der Industrieroboter (12, 12a-12g) die Sensorvorrichtung (26; 26a, 26b) und/oder die Reinigungsvorrichtung (32) trägt.

11. Reinigungssystem (10; 10a-10e), das Folgendes umfasst:
- das Steuersystem (18) nach Anspruch 10;
- den mindestens einen Industrieroboter (12; 12a-12g);
- die Sensorvorrichtung (26; 26a, 26b); und
- die Reinigungsvorrichtung (32).

12. Reinigungssystem (10; 10a-10e) nach Anspruch 10, wobei einer des mindestens einen Industrieroboters (12; 12a-12g) ein mobiler Roboter (12a-12c; 12f, 12g) mit einer Traktionsanordnung (24) ist und wobei die Reinigungsvorrichtung (32) von dem mobilen Roboter (12a; 12c; 12f, 12g) getragen wird.

13. Reinigungssystem (10; 10a-10e) nach Anspruch 12, das ferner eine Andockstation (40; 40a-40c) umfasst, wobei der mobile Roboter (12a; 12c; 12f, 12g) und die Andockstation (40; 40a-40c) so ausgebildet sind, dass der mindestens eine mobile Roboter (12a; 12c; 12f, 12g) an die Andockstation (40; 40a-40c) andocken und Reinigungsmedien (34a, 34b) von dort empfangen kann.

## Revendications

1. Procédé de nettoyage d'une région (52) dans un environnement industriel (48) à l'aide d'au moins un robot industriel (12 ; 12a-12g), le procédé consistant :
- à obtenir un rayonnement (28) provenant de la région (52) au moyen d'un dispositif capteur (26 ; 26a, 26b) ;
- à fournir des données de capteur (30) sur la base du rayonnement (28), les données de capteur (30) indiquant une propreté d'une ou de plusieurs surfaces (60a-60c) dans la région (52) ; et
- à réaliser un processus de nettoyage pour nettoyer la région (52), le processus de nettoyage consistant à appliquer des milieux de nettoyage (34a, 34b) à partir d'un dispositif de nettoyage (32) auxdites une ou plusieurs surfaces (60a-60c), et le processus de nettoyage étant réalisé sur la base des données de capteur (30), dans lequel le robot industriel (12, 12a-12g) porte le dispositif capteur (26 ; 26a, 26b) et/ou le dispositif de nettoyage (32).

2. Procédé selon la revendication 1, consistant en outre à déterminer, sur la base des données de capteur (30), si le processus de nettoyage doit être réalisé.

3. Procédé selon l'une quelconque des revendications précédentes, consistant en outre :
- à obtenir un rayonnement de validation (28) provenant de la région (52) au moyen du dispositif capteur (26 ; 26a, 26b) ;
- à générer d'autres données de capteur (30) sur la base du rayonnement de validation (28) ; et
- à évaluer le processus de nettoyage sur la base des données de capteur de validation (30).

4. Procédé selon l'une quelconque des revendications précédentes, consistant en outre à sélectionner un type de milieux de nettoyage (34a, 34b) à appliquer pendant le processus de nettoyage sur la base des données de capteur (30).

5. Procédé selon l'une quelconque des revendications précédentes, consistant en outre :
- à déterminer une distribution de contaminants (58 ; 58a, 58b) dans la région (52) sur la base des données de capteur (30) ; et
- à réaliser le processus de nettoyage sur la base de la distribution.

6. Procédé selon l'une quelconque des revendications précédentes, consistant en outre :
- à évaluer une propriété de surface desdites une ou plusieurs surfaces (60a-60c) dans la région (52) sur la base des données de capteur (30) ; et
- à réaliser le processus de nettoyage sur la base de la propriété de surface.

7. Procédé selon l'une quelconque des revendications précédentes, dans lequel l'un dudit au moins un robot industriel (12 ; 12a-12g) est un robot mobile (12a-12c ; 12f, 12g) comportant un agencement de traction (24).

8. Procédé selon la revendication 7, consistant en outre :
- à commander l'agencement de traction (24) pour déplacer le robot mobile (12a ; 12c ; 12f, 12g) afin qu'il s'arrime à une station d'accueil (40 ; 40a-40c) ; et
- à alimenter en milieux de nettoyage (34a, 34b) un ou plusieurs réservoirs (38a, 38b) du robot mobile (12a ; 12c ; 12f, 12g) à partir de la station d'accueil (40 ; 40a-40c)

9. Procédé selon l'une quelconque des revendications précédentes, consistant en outre à communiquer des données de gestion (46) à un système de gestion à distance (44), les données de gestion (46) étant associées aux données de capteur (30) et/ou associées au processus de nettoyage.

10. Système de commande (18) pour commander le nettoyage d'une région (52) dans un environnement industriel (48) à l'aide d'au moins un robot industriel (12 ; 12a-12g), le système de commande (18) comprenant au moins un dispositif de traitement de données (20) et au moins une mémoire (22) sur laquelle est stocké au moins un programme informatique, ledit au moins un programme informatique comprenant un code de programme qui, lorsqu'il est exécuté par ledit au moins un dispositif de traitement de données (20), amène ledit au moins un dispositif de traitement de données (20) à réaliser les étapes consistant :
- à ordonner à un dispositif capteur (26 ; 26a, 26b) d'obtenir un rayonnement (28) provenant de la région (52) ;
- à fournir des données de capteur (30) sur la base du rayonnement (28), les données de capteur (30) indiquant une propreté d'une ou de plusieurs surfaces (60a-60c) dans la région (52) ; et
- à commander la réalisation d'un processus de nettoyage pour nettoyer la région (52), le processus de nettoyage consistant à appliquer des milieux de nettoyage (34a, 34b) à partir d'un dispositif de nettoyage (32) auxdites une ou plusieurs surfaces (60a-60c), et le processus de nettoyage étant réalisé sur la base des données de capteur (34a), dans lequel le robot industriel (12, 12a-12g) porte le dispositif capteur (26 ; 26a, 26b) et/ou le dispositif de nettoyage (32).

11. Système de nettoyage (10 ; 10a-10e) comprenant :
- le système de commande (18) selon la revendication 10 ;
- ledit au moins un robot industriel (12 ; 12a-12g) ;
- le dispositif capteur (26 ; 26a, 26b) ; et
- le dispositif de nettoyage (32).

12. Système de nettoyage (10 ; 10a-10e) selon la revendication 10, dans lequel l'un dudit au moins un robot industriel (12 ; 12a-12g) est un robot mobile (12a-12c ; 12f, 12g) comportant un agencement de traction (24), et dans lequel le dispositif de nettoyage (32) est porté par le robot mobile (12a ; 12c ; 12f, 12g).

13. Système de nettoyage (10 ; 10a-10e) selon la revendication 12, comprenant en outre une station d'accueil (40 ; 40a-40c), dans lequel le robot mobile (12a ; 12c ; 12f, 12g) et la station d'accueil (40 ; 40a-40c) sont configurés de telle sorte que ledit au moins un robot mobile (12a ; 12c ; 12f, 12g) puisse s'arrimer à la station d'accueil (40 ; 40a-40c) et recevoir des milieux de nettoyage (34a, 34b) en provenance de celle-ci.
